(19) Europäisches Patentamt
European Patent Office
Office européen des brevets

(11) EP 0 729 344 B1

(12) **EUROPEAN PATENT SPECIFICATION**

(45) Date of publication and mention of the grant of the patent:
**28.08.2002 Bulletin 2002/35**

(21) Application number: **94929306.2**

(22) Date of filing: **22.09.1994**

(51) Int Cl.[7]: **A61K 7/06**, A61K 7/32, C07C 219/08, C07C 229/16, C07C 219/06, C11D 3/00, C11D 3/50

(86) International application number:
**PCT/US94/10748**

(87) International publication number:
**WO 95/008976 (06.04.1995 Gazette 1995/15)**

(54) **ACTIVE SUBSTANCE DELIVERY SYSTEM**

WIRKSTOFFABGABESYSTEM

SYSTEME FOURNISSANT UNE SUBSTANCE ACTIVE

(84) Designated Contracting States:
**DE ES FR GB IT**

(30) Priority: **30.09.1993 EP 93870196**

(43) Date of publication of application:
**04.09.1996 Bulletin 1996/36**

(73) Proprietor: **THE PROCTER & GAMBLE COMPANY**
**Cincinnati, Ohio 45202 (US)**

(72) Inventors:
- **USON, Isabel, Maria**
  **D-37083 Gottingen (DE)**
- **DEMEYERE, Hugo, Jean**
  **B-1881 Merchtem (BE)**
- **HARTMAN, Frederick, Anthony**
  **Cincinnati, OH 45242 (US)**
- **SIVIK, Mark, Robert**
  **Fairfield, OH 45242 (US)**

(74) Representative: **Engisch, Gautier et al**
**Procter & Gamble**
**European Technical Center N.V.**
**Temselaan 100**
**1853 Strombeek-Bever (BE)**

(56) References cited:

| | |
|---|---|
| **WO-A-87/06826** | **DE-A- 2 947 775** |
| **DE-A- 3 734 185** | **US-A- 3 989 711** |
| **US-A- 4 429 859** | **US-A- 4 767 547** |

- **PATENT ABSTRACTS OF JAPAN vol. 15, no. 133 (C-0820), 2 April 1991 & JP-A-03 017025 (NIPPON OIL & FATS CO LTD)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 729 344 B1

## Description

The present invention relates to compositions comprising a softening conditions and a compound containing a nitrogen linked by an ester bond to an active substance radical. Said compositions show an excellent deposition of the active substance radical to a surface followed by delayed release of an active substance to the surface.

It is well known that by using active substances such as alcohols and/or acids often the substances show a difficultly controllable activity due to their inherent characteristics. Although the present invention is not limited to fabric softener compositions, but for instance when using fabric softener compositions, one provides the usual compositions with additional perfumes, antiperspirant compounds etc. in order to obtain the desired result after the rinse cycle of laundry washing processes.

In recent years, the need has arisen for more environmentally-friendly materials, and rapidly biodegradable quaternary ammonium compounds have been presented as alternatives to the traditionally used di-long chain ammonium chlorides. Such quaternary ammonium compounds contain long chain alk(en)yl groups interrupted by functional groups such as carboxy groups.

Said materials and fabric softening compositions containing them are disclosed in numerous publications such as EPA 0 040 562, and EPA 239 910;

In EPA 239 910, it has been disclosed that a pH range of from 2.5 to 4.2 provides optimum storage stability to said rapidly biodegradable ammonium compounds.

A disadvantage of above system is the difficulty to control the activity of the active substances such as for instance the perfumes and antiperspirants in the respective softener composition.

Active substances have a poor affinity for surfaces. Once on a surface they are removed due to their water solubility or high volatility and might sometimes lose all of their activity.

In EP 56152 is described a process for the simultaneous softening and antimicrobial finishing of washed laundry in an aqueous liquor containing fabric-softener quaternary ammonium compounds and antimicrobially active substances by allowing the liquor to act on items of laundry, separating off the liquor from the items of laundry and drying the laundry. The items of laundry are moved around in a liquor which, in addition to at least one standard fabric-softening quaternary compound, contains at least one azole compound as the antimicrobially active substance.

However in this system as described in EP 56152 the activity of the active substance is difficult to control in order to obtain the best desirable results in terms of for instance malodour prevention.

US-A-4,429,859 discloses concentrated softening compositions comprising a softening compound.

US-A-4,767,547 discloses biodegradable fabric softeners.

US-A-3,989,711 discloses soft quaternary surface active agents exhibiting antibacterial activity.

DE-A-2,947,775 discloses derivatives of farnesyl and their use in pharmacy.

WO-A-87/06826 discloses imidazolinium compounds for softening and conditioning fibers.

JP-A-03-017025 discloses a sustained release agent for active component composed of an ester of an oxyalkylene derivative.

A solution to above-mentioned problem is that the present invention allows to formulate a composition, and in a preferred embodiment a fabric softener composition, wherein the basic principle is to link an active substance such as an alcohol or an acid to a nitrogen via an ester bond.

Active substances which in themselves are not surface substantive are by using the present invention efficiently deposited on the surface. Probably as a consequence of the weak ester linkage the release of the active substance is induced for instance by a hydrolysis process. When the surface is a fabric this development allows surprisingly effective deposition and retention of active substances on fabrics, followed by a slow release of the active substance over time.

The active substance might be an acid or an alcohol. The active is bound via an ester linkage to an alcohol or acid function in the central part of a compound according to the invention wherein the compound has the structural formula:

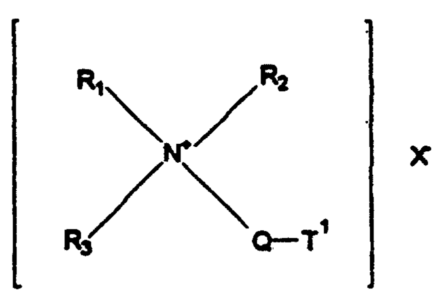

or an amine precursor thereof
wherein :

$R_1$ is Q'-$T^1$ or Q'-$T^2$ or $T^2$ or $R_6$,
$R_2$ is Q"-$T^1$ or Q"-$T^3$ or $T^3$ or $R_6$,
Q is

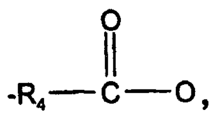, 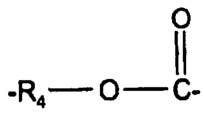, or 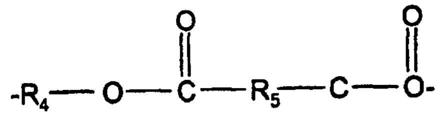,

Q' and Q" are defined as Q wherein $R_4$ is $R_4$' or $R_4$" and $R_5$ is $R_{5'}$ or $R_{5''}$ respectively
$R_3$ and $R_6$ are the same or different $C_1$-$C_4$ alkyl or $C_1$-$C_4$ hydroxyalkyl or H
$R_4$, $R_{4'}$ and $R_{4''}$ and $R_5$, $R_{5'}$ and $R_{5''}$ are any acyclic or any mono or poly (hetero)cyclic organic group,
$T^1$ is an active substance radical,
$T^2$ and $T^3$ are (the same or different) $C_5$-$C_{30}$ alkyl or alkenyl or aryl, $X^-$ is a compatible anion.

$T^1$ is defined as an active substance radical. With "active substance radical" is meant that part of the compound which, for instance, upon hydrolysis generates the active substance.

If the active substance is an alcohol then the alcohol active may first be reacted with a diacid (or diacid anhydride) to form an acid ester. This product is then linked via the free acid function to the free alcohol function in the central part of the compound for use according to the invention. Alternatively, the alcohol active is linked directly to a free acid function in the central part of the compound.

The central part in the molecule can be any ethoxylated amine (e.g. triethanolamine or methyldiethanolamine) or it could be a more complex hydroxyfunctionalized amine or quat or a (poly)carboxyfunctionalized amine or quat.

The active substance may be any acid or alcohol one wants to deposit on fabrics. Depending on the application, one can use a monoester, a mixed ester with fatty acid or a polyester of the active substance with the central part of the compound

This system may also be used to deposit an active substance on another substrate than fabrics, e.g. skin, hair or any other surface that one wants to treat.

One application is in perfume delivery in a fabric softener context.

This technology allows very efficient deposition of a perfume ingredient followed by slow release upon hydrolysis of the ester bond linking the active substance to the central part of the molecule.

Other potential applications in a fabric softener matrix are biocide delivery (malodour prevention), antiperspirant delivery (in wear malodour prevention) and iron-aid delivery.

This delivery system might depending on the choice of the active substance also find an application outside a fabric softener context: e.g. perfume delivery in a detergent, antiperspirant delivery in a cosmetic product for instance in a deodorant, moldicide delivery in an anti-dandruff shampoo, top note delivery during ironing by release of the volatile top notes during ironing by humidity and/or heat induced hydrolysis, in an ironing-aid, pesticide, or insecticide/insect repellent delivery in a pesticide or corrosion inhibition delivery in a hard surface cleaner.

## Detailed description of the invention

Part of the present invention is a composition wherein the compound has a nitrogen present in a quaternary ammonium group and/or amine precursor thereof.

The compound could have the following structural formulas:

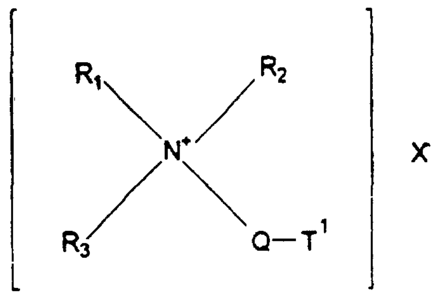

wherein :

$R_1$ is Q'-$T^1$ or Q'-$T^2$ or $T^2$ or $R_6$,
$R_2$ is Q"-$T^1$ or Q"-$T^3$ or $T^3$ or $R_6$,
Q is

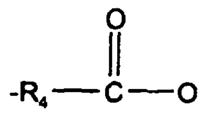, 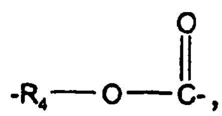, or 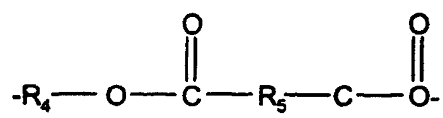,

Q' and Q" are defined as Q wherein $R_4$ is $R_{4'}$ or $R_{4''}$ and $R_5$ is $R_{5'}$ or $R_{5''}$ respectively,
$R_3$ and $R_6$ are the same or different $C_1$-$C_4$ alkyl or $C_1$-$C_4$ hydroxyalkyl or H,
$R_4$, $R_{4'}$ and $R_{4''}$ and $R_5$, $R_{5'}$ and $R_{5''}$ are any acyclic or any mono or poly (hetero)cyclic organic group,
$T^1$ is an active substance radical,
$T^2$ and $T^3$ are (the same or different) $C_5$-$C_{30}$ alkyl or alkenyl or aryl, $X^-$ is a compatible anion.

The anion is merely present as a counterion of the positively charged quaternary ammonium compounds or protonated amines. The scope of this invention is not considered limited to any particular anion, although the use of some particular anion may be preferred in certain compositions.

By "amine precursors thereof" is meant the secondary or tertiary amines corresponding to the above quaternary ammonium compounds.

The present invention also encompasses a composition comprising a compound containing a nitrogen wherein this nitrogen is present in an imidazolium, imidazolinium or pyridinium group or another cyclic ammonium compound and/or precursor thereof or in an amineoxide group. The compound could have the following structural formulas:

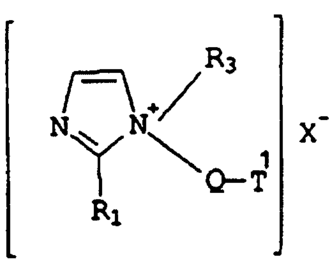 or 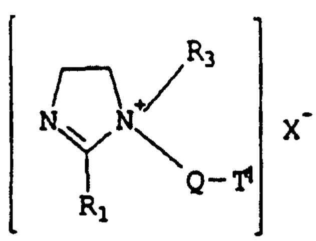 or 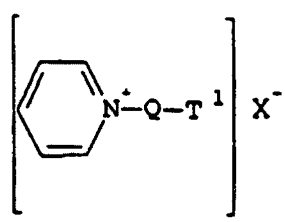

or

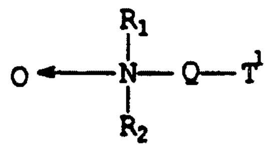

wherein:

$R_1$ is Q'-$T^1$ or Q'-$T^2$ or $T^2$ or $R_{6,}$
$R_2$ is Q"-$T^1$ or Q"-$T^3$ or $T^3$ or $R_{6,}$
Q is

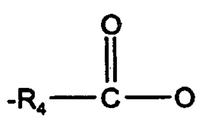, 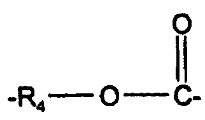, or 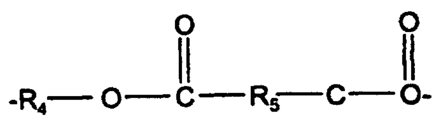,

Q' and Q" are defined as Q wherein $R_4$ is $R_{4'}$ or $R_{4''}$ and $R_5$ is $R_{5'}$ or $R_{5''}$ respectively,
$R_3$ and $R_6$ are the same or different $C_1$-$C_4$ alkyl or $C_1$-$C_4$ hydroxyalkyl or H,
$R_4$, $R_{4'}$ and $R_{4''}$ and $R_{5,}$ $R_{5'}$ and $R_{5''}$ are any acyclic or any mono or poly (hetero)cyclic organic group,
$T^1$ is an active substance radical,
$T^2$ and $T^3$ are (the same or different) $C_5$-$C_{30}$ alkyl or alkenyl or aryl, $X^-$ is a compatible anion.

Part of the invention is also a composition comprising a compound containing several nitrogens wherein the compound has the following structural formulas:

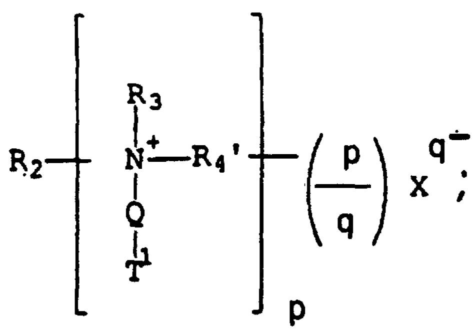; or 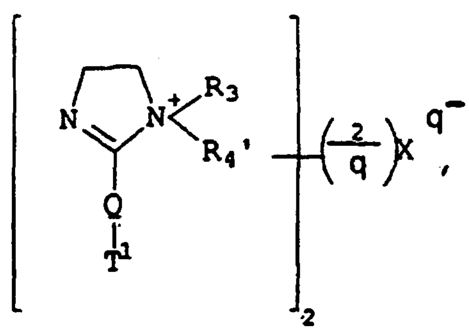,

or

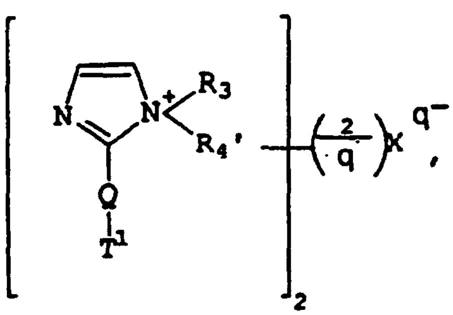, or 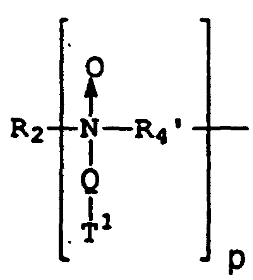

or an amine precursor thereof
wherein :

$R_2$ is $Q''$-$T^1$ or $Q''$-$T^3$ or $T^3$ or $R_6$,
Q is

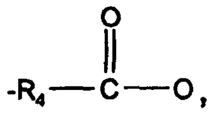, 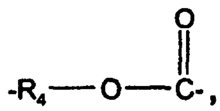, or 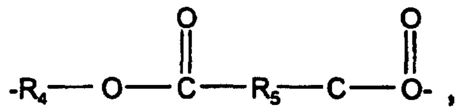,

Q" is defined as Q wherein $R_4$ is $R_{4''}$ and $R_5$ is $R_{5''}$.
$R_3$ is $R_6$ are the same or different $C_1$-$C_4$ alkyl or $C_1$-$C_4$ hydroxyalkyl or H,
$R_4$, $R_{4'}$, $R_{4''}$, $R_5$ and $R_{5''}$ are any acyclic or any mono or poly (hetero)cyclic organic group,
$T^1$ is an active substance radical,
$T^2$ and $T^3$ are (the same or different) $C_5$-$C_{30}$ alkyl or alkenyl or aryl, $X^-$ is a compatible anion, p and q are integer numbers.

Also part of the invention is a composition comprising a compound with any of the following structural formulas :

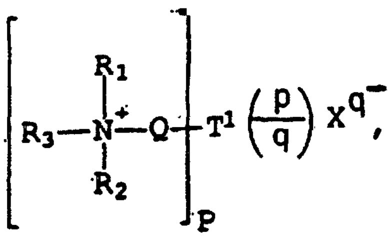, or 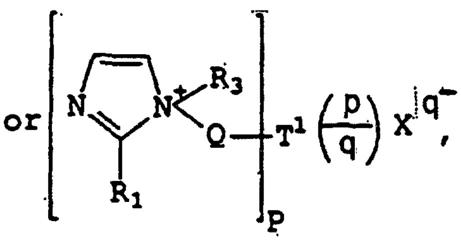,

or

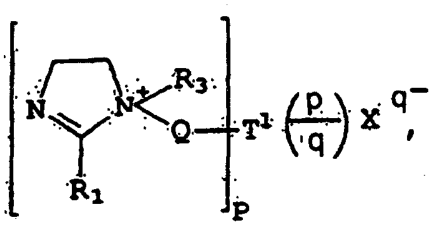, or 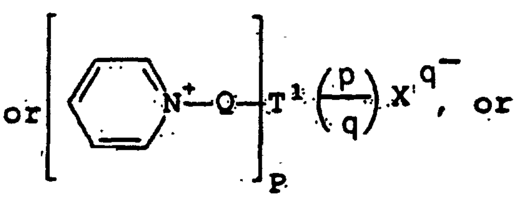, or 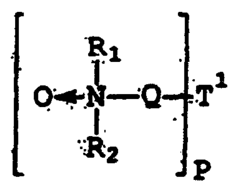

or an amine precursor thereof
wherein:

$R_1$ is $Q'$-$T^1$ or $Q'$-$T^2$ or $T^2$ or $R_6$,
$R_2$ is $Q''$-$T^1$ or $Q''$-$T^3$ or $T^3$ or $R_6$
Q is

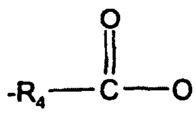 , 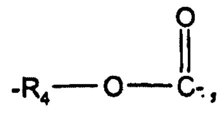 , or 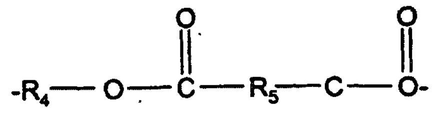 ,

Q' and Q" are defined as Q wherein $R_4$ is $R_{4'}$ or $R_{4''}$ and $R_5$ is $R_{5'}$ or $R_{5''}$ respectively,
$R_3$ and $R_6$ are the same or different $C_1$-$C_4$ alkyl or $C_1$-$C_4$ hydroxyalkyl or H,
$R_4$, $R_{4'}$ and $R_{4''}$ and $R_5$, $R_{5'}$ and $R_{5''}$ are any acyclic or any mono or poly (hetero)cyclic organic group,
$T^1$ is a polyhydroxy and/or polycarboxy functional active substance radical,
$T^2$ and $T^3$ are (the same or different) $C_5$-$C_{30}$ alkyl or alkenyl or aryl, $X^-$ is a compatible anion
p is an integer number between 1 and $\frac{MWT^1}{50}$, preferably between $\frac{MWT^1}{1000}$ and $\frac{MWT^1}{100}$, and q is an integer number.

The active substance according to the invention can be selected from the class of active substances comprising acids or alcohols of natural or synthetic origin.

Furthermore the active substance according to the present invention can be selected from hydroxy or carboxy functional perfume ingredients, bactericides, fungicides, antiperspirants, starch or modified starch, pesticides, insecticides, insect repellants or corrosion inhibitors.

Depending on the application wherein the present composition according to the invention can be used additionally, for instance, conventional fabric conditioner or fabric softener matrix components and additives can be added to the composition.

In another application additionally conventional detergent matrix components and additives can be added.

Also conventional perfume and/or deodorizing matrix components, conventional shampoo/hair conditioner matrix components, pesticide matrix components, or hard-surface cleaner matrix components, can be added to the composition according to the invention.

Depending on the active substance chosen and the respective matrix components the composition can be used as a perfume delivery system, as a biocide delivery system, as a fungicide delivery system, as an antiperspirant delivery system, as an insecticide or insect repellent system or as an iron-aid delivery system.

Ingredients to be used in order to obtain a fabric softener composition are for instance described in EP 239,910.

## EXAMPLES

The following examples illustrate the present invention.

### Example 1

Synthesis of a compound according to the invention wherein the active substance is geranic acid.

1 mole of geranic acid is reacted with 1 mole of methyldiethanolamine (160°C, 16 h, vacuum) to form the ester of the geranic acid with methyldiethanolamine. The reaction mixture is further reacted with 1 mole of stearic acid (160°C, 24 h, vacuum) to form the mixed diester with methyldiethanolamine.
The diester is then quaternized using methylchloride adding 15% of isopropanol as quaternizing solvent.
The resulting quaternary ammonium compound can be incorporated into a composition according to the present invention.

### Example 2

Synthesis of a compound according to the invention wherein the active substance is citronellol.

1 mole of N-methyl, N,N-diacetic acid amine is reacted with 0.5 moles of citronellol(150°C, 24 h, vacuum). The resulting mixture is further reacted with 1.5 mole of fatty alcohol (150°C, 24 h, vacuum ). The diester is then quaternized using methylchloride adding 10% of isopropanol as quaternizing solvent. The resulting quaternary ammonium compound can be incorporated into a composition according to the present invention.

### Example 3

Synthesis of a compound according to the invention wherein the active substance is isovaleric acid. Assessment of the improved activity.

3.a) Chemical synthesis

1 mole methyldiethanolamine was reacted with 0.2 moles of isovaleric acid (160 °C, 16 h, vacuum) to form the monoester of isovaleric acid and methyldiethanolamine.
The reaction mixture was further reacted with 1.8 moles of stearic acid (160 °C, 24 h, vacuum) to form the mixed diester. The diester was then quaternized using methylchloride adding 15% of isopropanol as quaternizing solvent. 1000 g of this raw material thus contains 850 g of quaternary ammonium compound and 27.3 g of isovaleric acid chemically bound to the quaternary ammonium compound.

3.b) Dispersion making

A 7% acidified dispersion of the under 1.a described raw material was prepared.

1) 927.3 g of demineralised water was heated to 65°C and kept at that temperature.
2) 2.7 g of a 10% hydrochloric acid solution was added to the water.
3) 70 gr of the raw material described under 1.a was heated to 75°C and added to the acidified water under continuous agitation. Addition time was 1 minute.
4) The resulting dispersion was cooled to ambient temperature under continuous agitation.

The dispersion should have a 10% pH of 3.3 ± 0.3.
The finished product contains 0.19% of isovaleric acid chemically bound to the quaternary ammonium compound.

3.c) Product application

2.1 kg of fabrics (60% pure cotton, 40% mixed polyester/cotton) were washed in a AEG öko-lavamat 579 at 60°C (mainwash only) using 180 g of regular Belgium Ariel® detergent.
The load contained 12 terry (cotton) tracers of 30 g each.

3.c.1 Reference treatment

A reference dispersion was prepared according to the procedure described under 1.b, using 70 g of standard quaternary ammonium compound (i.e. quaternary ammonium compound synthesized as described in 1.a, without addition of isovaleric acid and using 2 moles of stearic acid) to which 2.73% of isovaleric acid was admixed.
This reference dispersion thus contains 0.19% of free isovaleric acid.

3.c.1.a (Free active, no extra rinse)

110 g of this reference dispersion was added to the last rinse of the washing machine and 6 of the 12 tracers were removed after the machine had stopped the rinse cycle.

3.c.1.b (Free active, extra rinse)

After the removal of the first 6 tracers, the remaining load was subjected to an extra rinse cycle in the same washing machine. The remaining 6 tracers were removed after the machine had stopped the rinse cycle.

3.c.2) Test treatment

3.c.2.a (EQDS, no extra rinse)

110 g of the dispersion described under 1.b was added to the last rinse of the washing machine and 6 of the 12 tracers were removed after the machine had stopped the rinse cycle.

3.c.2.b (EQDS, extra rinse)

After the removal of the first 6 tracers, the remaining load was subjected to an extra rinse cycle in the same washing machine. The remaining 6 tracers were removed after the machine had stopped the rinse cycle.

3.d) Fabric grading

3.d.1) Tracer storage

The tracers were graded as they were removed from the washing machine (damp). The fabrics were then line dried at ambient temperature and again graded the following day (1 day dry). The tracers were then packed in aluminium foil and stored on a lab bench at ambient temperature. The judges reevaluated after 1 week and 2 weeks storage.

3.d.2) Grading procedure

The tracers were compared in pairs (free, no extra rinse vs EQDS, no extra rinse; free, extra rinse vs EQDS, extra rinse). The pairs were graded blindly by 4 judges. The tracers were presented to the judges in alternating order. The judges used the following scale:

0: There is no difference
1: I think one smells stronger
2: I know one smells stronger
3: I know one smells much stronger
4: One smells a whole lot stronger.

The test supervisor gave a positive sign to the grade when the EQDS treatment scored stronger, a negative sign when the reference treatment scored stronger.
The average of the different gradings is reported.

3.d.3) Results

3.d.3.a) No extra rinse

EQDS vs free isovaleric acid, + indicates stronger smell for EQDS, 0-4 scale.

| | |
|---|---|
| Damp | -1.5 |
| 1 day dry | 0 |
| 1 week storage | 2.5 |
| 2 weeks storage | 3.0 |

3.d.3.b) With extra rinse

EQDS vs free isovaleric acid, + indicates stronger smell for EQDS, 0-4 scale.

| | |
|---|---|
| Damp | 0 |
| 1 day dry | 1.5 |
| 1 week storage | 2.5 |
| 2 weeks storage | 3.0 |

Above mentioned results clearly indicate that the active radical has been effectively deposited to fabrics, was retained on the fabrics during a subsequent rinse and that the active substance was gradually released during fabric storage.

**Example 4**

The following fabric softener composition containing the compound according to example 1 further illustrates the present invention.

| | |
|---|---|
| N,N-di(2-tallowoxyl-oxy-ethyl) N,N-dimethyl-ammoniumchloride | 18% |
| Tallow Alcohol 25 ethoxylate | 2% |

(continued)

| | |
|---|---|
| PolyGlycerolMonoStearate | 5.0% |
| HCl | 0.08% |
| CaCl2 | 0.3% |
| Perfume | 0.5% |
| Compound according to ex. 1 | 0.3% |
| Dye, antifoam, water | Balance |

**Example 5**

The following detergent composition containing the compound according to example 1 further illustrates the present invention.

| | |
|---|---|
| C11-12 AlkylBenzeneSulfonate (Na) | 6.5% |
| Tallow Alcohol sulfate (Na) | 1.0% |
| Tallow alcohol ethoxylate (EO11) | 0.8% |
| Hydrogenated tallow fatty acid | 1.0% |
| Distearyl-methyl-amine | 3.0% |
| Dodecyl dimethyl ammonium N-oxide | 0.4% |
| Zeolite | 20% |
| polyethyleneoxide (MW=5MM) | 0.05% |
| Sodium sulfate | 12.7% |
| Sodium silicate | 2.0% |
| Sodium perborate (4 aq.) | 20.0% |
| Carboxymethylcellulose | 0.4% |
| Polyacrylate (MW=4000-5000) | 3.0% |
| Enzymes (proteases, amylase, cellulase) | 0.3% |
| Optical brightener | 0.25% |
| Ethylenediamine-tetramethylene-phosphonic acid | 0.1% |
| Tetraacetyl-ethylenediamine | 1.5% |
| Silicone/silica suds suppressor | 0.2% |
| Montmorillonite clay | 10% |
| Perfume | 0.2% |
| Compound of example 1 | 0.5% |

**Example 6**

The following hair conditioner composition containing the compound according to example 1 further illustrates the present invention.

| | |
|---|---|
| Tallowalcohol ethoxylate (TAE11) | 2.0% |
| Dimethyl-benzyl-stearyl-ammonium chloride | 1.0% |
| CetylMonoGlyceride | 2.0% |
| Cetyl Alcohol | 2.5% |
| Methyl-Hydroxypropyl-cellulose | 0.7% |
| Quaternized hydroxyethylcellulose | 0.5% |
| Hydrochloric acid | 0.2% |
| Perfume | 0.2% |
| Compound according to ex. 1 | 0.3% |
| Dye, preservative, water | Balance |

## Claims

1. A composition comprising a softening conditioner and a compound containing a nitrogen linked by an ester bond to an active substance radical $T^1$,

   wherein the active substance radical generates an active substance selected from the class of acids or alcohols of natural or synthetic origin,

   wherein the active substance being active in such a way that the active substance is effectively deposited on a surface and being effectively retained on said surface, followed by delayed release over time,

   wherein the softening conditioner is selected from the group consisting of fabric softener matrix components and additives, detergent matrix components and additives, shampoo matrix components and additives, hair conditioner matrix components and additives, hard surface cleaner matrix components and additives, perfume matrix components and additives, deodorant matrix components and additives or insecticide matrix components and additives, and

   wherein $T^1$ will generate an active substance selected from the class of hydroxy and/or carboxy functional perfume ingredients, bactericides, fungicides, anti perspirants, starch or modified starch, pesticides, insecticides, insect repellants and corrosion inhibitors.

2. A composition according to claim 1 wherein the compound has a nitrogen present in a quaternary ammonium group and/or an amine precursor thereof.

3. A composition according to claim 2 wherein the compound has the following structural formula:

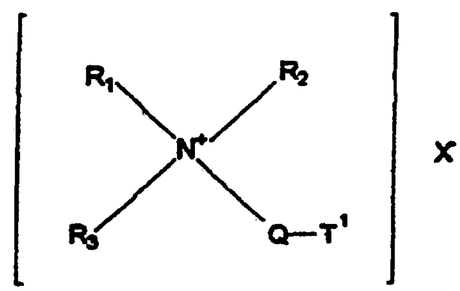

   or an amine precursor thereof

   wherein :

   $R_1$ is Q'-$T^1$ or Q'-$T^2$ or $T^2$ or $R_6$,
   $R_2$ is Q"-$T^1$ or Q"-$T^3$ or $T^3$ or $R_6$,
   Q is

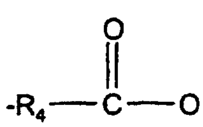,  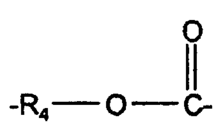,  or  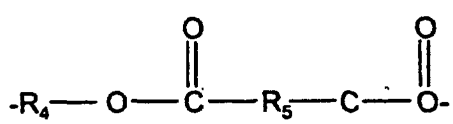,

   Q' and Q" are defined as Q wherein $R_4$ is $R_{4'}$ or $R_{4''}$ and $R_5$ is $R_{5'}$ or $R_{5''}$ respectively,
   $R_3$ and $R_6$ are the same or different $C_1$-$C_4$ alkyl or $C_1$-$C_4$ hydroxyalkyl or H;
   $R_4$- $R_{4'}$ and $R_{4''}$ and $R_5$, $R_{5'}$ and $R_{5''}$ are any acyclic or any mono or poly (hetero) cyclic organic group,
   $T^1$ is the active substance radical
   $T^2$ and $T^3$ are (the same or different) $C_5$-$C_{30}$ alkyl or alkenyl or aryl, $X^-$ is a compatible anion.

4. A composition according to claim 3 wherein $R_4$ is $(CH_2)_n$ or $(CH_2\text{-}CH_2\text{-}O)_nCH_2\text{-}CH_2\text{-}$ and $R_5$ is $C_1$-$C_4$ alkyl or alkenyl or aryl and n is an integer between 1 and 4.

5. A composition according to claim 3 wherein $R_4$ contains one or more hydroxy and/or carboxy and/or ester functions.

6. A composition according to claim 3 wherein the compound is:

$$[\ N\ (Me)x\ (CH_2\text{-}CH_2\text{-}OOC\text{-}T_4)y(CH_2\text{-}CH_2\text{-}O\text{-}R_7)z]+\ X\text{-}$$

or

$$[\ N\ (Me)x\ ((CH_2)_n\text{-}COO\text{-}T_1)y((CH_2)_n\text{-}COO\text{-}R_8)Z]+\ X\text{-}$$

or

$$[\ N\ (Me)3\ \text{-}\ (CH_2\text{-}CH(OOC\text{-}T_4)(CH_2\text{-}O\text{-}R_9))]+\ X\text{-}$$

or

$$[\ N\ (Me)\ 3\ \text{-}\ (CH_2\text{-}CH(OR_9)\ (CH_2\text{-}OOC\text{-}T_4))]+\ X\text{-}$$

or an amine precursor of one of the above,
Where x+y+z = 4
n is an integer between 1 and 4
and $R_7$ is H or -OC-$T_2$ ; $R_8$ is H or $T_2$; $R_9$ is $R_7$ or - OC - $T_4$
T2 is defined as in claim 3,
T4 is T1 as defined in claim 3, or is an active substance radical that, upon hydrolysis, generates an active substance and a diacid and
X- is Chloride or MeSulfate,

7. A composition according to claim 1 comprising a compound containing a nitrogen, wherein the nitrogen atom in said compound is part of a cyclic ammonium moiety and/or of an amine precursor of said compound or of an amineoxide group.

8. A composition according to claim 7 wherein the compound has the following structural formula:

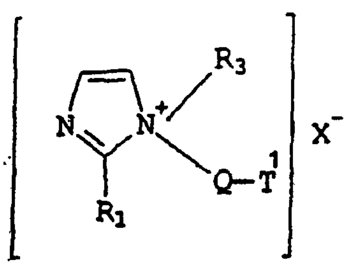

or

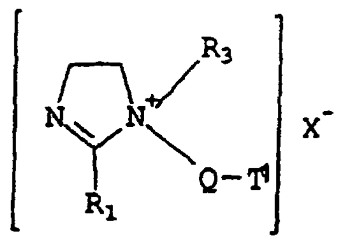

or

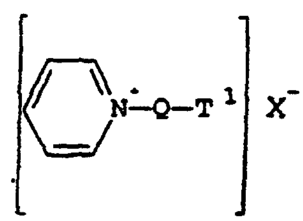

or an amine precursor thereof
or

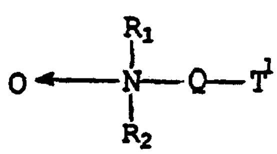

wherein :

$R_1$ is Q'-$T^1$ or Q'-$T^2$ or $T^2$ or $R_6$,
$R_2$ is Q"-$T^1$ or Q"-$T^3$ or $T^3$ or $R_6$,
Q is

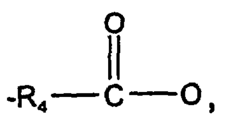, 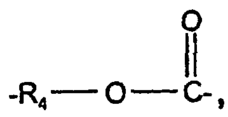, or 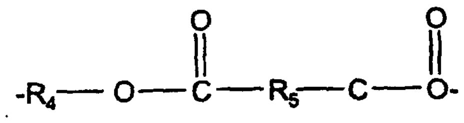,

Q' and Q" are defined as Q wherein $R_4$ is $R_{4'}$ or $R_{4''}$ and $R_5$ is $R_{5'}$ or $R_{5''}$ respectively,
$R_3$ and $R_6$ are the same or different $C_1$-$C_4$ alkyl or $C_1$-$C_4$ hydroxyalkyl or H,
$R_4$, $R_{4'}$ and $R_{4''}$ and $R_5$, $R_{5'}$ and $R_{5''}$ are any acyclic or any mono or poly (hetero)cyclic organic group,
$T^1$ is an active substance radical,
$T^2$ and $T^3$ are (the same or different) $C_5$-$C_{30}$ alkyl or alkenyl or aryl, $X^-$ is a compatible anion.

**9.** A composition according to claim 8 wherein $R_4$ is $(CH_2)_n$ or $(CH_2$-$CH_2$-$O)_nCH_2$-$CH_2$- and $R_5$ is $C_1$-$C_7$ alkyl or alkenyl or aryl and n is an integer between 1 and 4.

**10.** A composition according to claim 1 comprising a compound containing several nitrogens wherein the compound has the following structural formula:

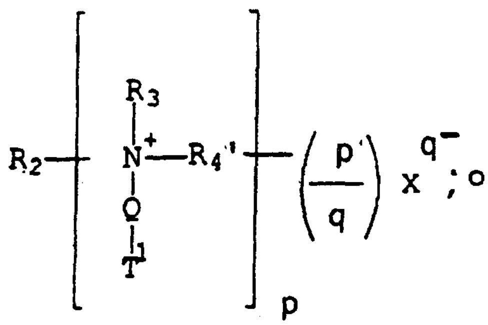 or 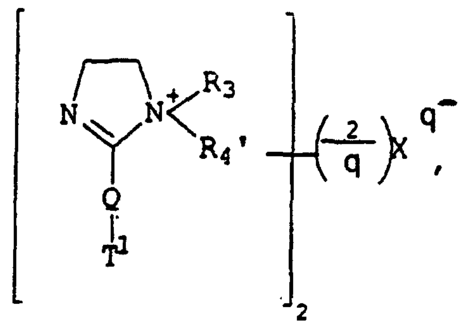

or

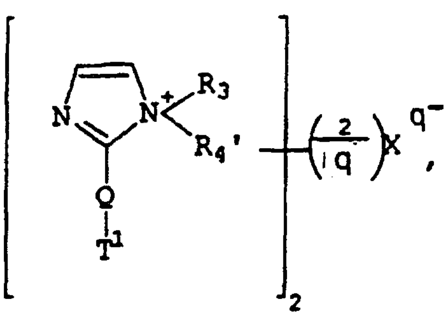, or 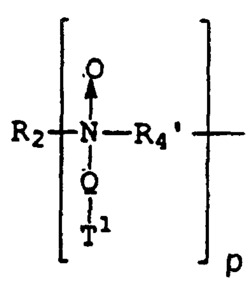

or an amine precursor thereof
wherein :

$R_2$ is Q"-$T^1$ or Q"-$T^3$ or $T^3$ or $R_6$.

Q is

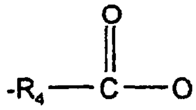, 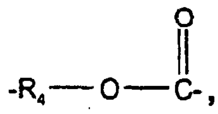, or 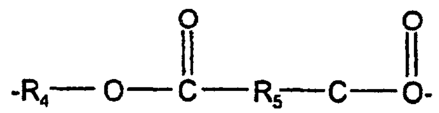,

Q" is defined as Q wherein $R_4$ is $R_{4''}$ and $R_5$ is $R_{5''}$,
$R_3$ and $R_6$ are the same or different $C_1$-$C_4$ alkyl or $C_1$-$C_4$ hydroxyalkyl or H,
$R_4$, $R_{4'}$, and $R_{4''}$, $R_5$, and $R_{5''}$ are any acyclic or any mono or poly (hetero)cyclic organic group,
$T^1$ is an active substance radical,
$T^3$ is $C_5$-$C_{30}$ alkyl or alkenyl or aryl, $X^-$ is a compatible anion.
p and q are integers.

**11.** A composition according to claim 10 wherein $R_4$ is $(CH_2)_n$ or $(CH_2\text{-}CH_2\text{-}O)_nCH_2\text{-}CH_2\text{-}$ and $R_5$ is $C_1$-$C_7$ alkyl or alkenyl or aryl and n is an integer between 1 and 4.

**12.** A composition according to claim 1, in which the compound containing nitrogen atoms has the following structural formula:

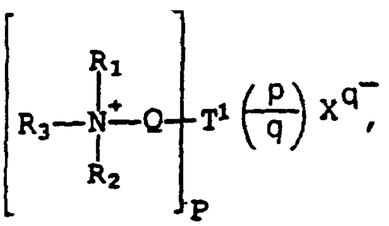, or 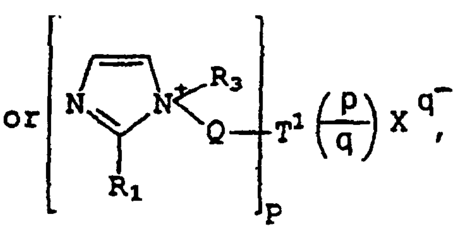,

or

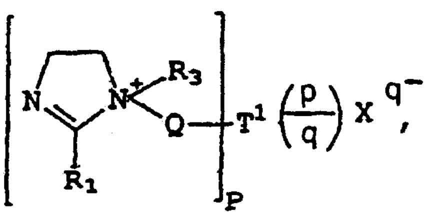, or 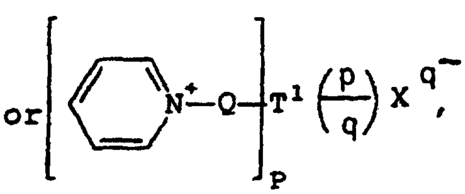, or 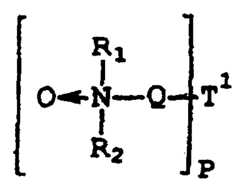

or an amine precursor thereof
wherein :

$R_1$ is Q'-$T^1$ or Q'-$T^2$ or $T^2$ or $R_6$,
$R_2$ is -Q"-$T^1$ or Q"-$T^3$ or $T^3$ or $R_6$,
Q is

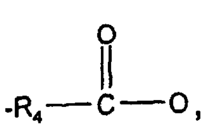, 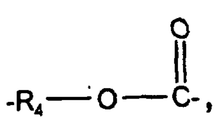, or 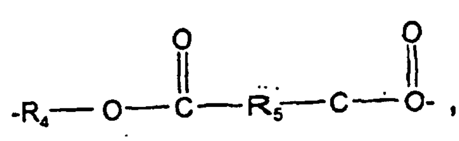,

Q' and Q" are defined as Q where in $R_4$ is $R_{4'}$ or $R_{4''}$ and $R_5$ is $R_{5'}$ or $R_{5''}$ respectively,
$R_3$ and $R_6$ are the same or different $C_1$-$C_4$ alkyl or $C_1$-$C_4$ hydroxyalkyl or H,
$R_4$, $R_{4'}$ and $R_{4''}$ and $R_5$, $R_{5'}$ and $R_{5''}$ are any acyclic or any mono or poly (hetero)cyclic organic group,
$T^1$ is an active substance radical,
$T^2$ and $T^3$ are (the same or different) $C_5$-$C_{30}$ alkyl or alkenyl or aryl, $X^-$ is a compatible anion.
p is an integer number between 1 and $\frac{MWT^1}{50}$, preferably between $\frac{MWT^1}{1000}$ and $\frac{MWT^1}{100}$ and q is an integer.

**13.** A composition according to claim 12 wherein $R_4$ is $(CH_2)_n$ or $(CH_2\text{-}CH_2\text{-}O)_nCH_2\text{-}CH_2\text{-}$ and $R_5$ is $C_1$-$C_7$ alkyl or alkenyl or aryl and n is an integer between 1 and 4.

**14.** A composition according to claims 3, 6, 8, 10, and 12 where $T^2$ and $T^3$ are (the same or different) $C_{12}$-$C_{22}$ alkyl or alkenyl.

**15.** A composition according to claim 1 which additionally contains fabric softener matrix components and additives selected from silicone, soil release agent, organic solvent, nonionics, electrolyte, perfume, preservation, germicides, colorants, dyes, fungicides, stabiliser, brightener, opacifin, and mixtures thereof.

**16.** A composition according to claim 15 containing between 0.005% and 20.0% of active substance radical, preferably between 0.01% and 5.0% and most preferably between 0.02% and 2.0% of active substance radical.

**17.** A composition according to claim 1 wherein $X^-$ is a $C_7$-$C_{30}$ alkyl or alkenyl containing anion.

**18.** Use of a composition comprising a softening conditioner and a compound containing a nitrogen linked by an ester bond to an active substance radical, the active substance radical generating an active substance selected from the clan of acids or alcohols of natural or synthetic origin; or a composition according to claim 15 in a fabric softener/conditioner matrix as a perfume delivery system, as a biocide delivery system, as a fungicide delivery system, as an antiperspirant delivery system, as an insecticide or insect repellent system or as an iron-aid delivery system.

**19.** Use of a composition comprising a softening conditioner and a compound containing a nitrogen linked by an ester bond to an active substance radical, the active substance radical generating an active substance selected from the clan of acids or alcohols of natural or synthetic origin; or a composition according to claim 1 in a detergent matrix as a perfume, biocide, fungicide, antiperspirant, insecticide or insect repellent or as an iron-aid delivery system.

**20.** Use of a composition comprising a softening conditioner and a compound containing a nitrogen linked by an ester bond to an active substance radical, the active substance radical generating an active substance selected from the clan of acids or alcohols of natural or synthetic origin; or a composition according to claim 1 as a perfume delivery system, a bactericide or insecticide/insect repellent delivery system, a fungicide delivery system, an antiperspirant delivery system, an anti-dandruff delivery system or a corrosion inhibitor delivery system.

**Patentansprüche**

**1.** Zusammensetzung, enthaltend ein weichmachendes Konditionierungsmittel und eine Verbindung, enthaltend einen Stickstoff, der durch eine Esterbindung an einen Wirksubstanzrest $T^1$ gebunden ist, worin der Wirksubstanzrest eine Wirksubstanz bildet, ausgewählt aus der Klasse der Säuren oder Alkohole aus natürlichem oder synthetischem Ursprung,
worin die Wirksubstanz in einer solchen Weise wirksam ist, dass die Wirksubstanz auf einer Oberfläche wirksam abgesetzt wird und wirksam auf dieser Oberfläche verbleibt, gefolgt von einer verzögerten Freisetzung im Laufe der Zeit,
worin das weichmachende Konditionierungsmittel ausgewählt ist aus der Gruppe, bestehend aus Gewebeweichmachermatrixkomponenten und -additiven, Reinigungsmittelmatrixkomponenten und -additiven, Shampoomatrixkomponenten und -additiven, Haarkonditionierungsmittelmatrixkomponenten und -additiven, Matrixkomponenten und Additive für Reiniger für harte Oberflächen, Duftstoffmatrixkomponenten und -additive, Deodorantmatrixkomponenten und -additive oder Insektizidmatrixkomponenten und -additive, und
worin $T^1$ eine Wirksubstanz bildet, ausgewählt aus der Klasse von hydroxy- und/oder carboxyfunktionalen Duftstoffbestandteilen, Bakteriziden, Fungiziden, Antitranspirationsmitteln, Stärke oder modifizierter Stärke, Pestiziden, Insektiziden, Insektenabwehrmitteln und Korrosionsinhibitoren.

2. Zusammensetzung nach Anspruch 1, worin die Verbindung einen Stickstoff in einer quaternären Ammoniumgruppe und/oder einer Aminvorstufe davon aufweist.

3. Zusammensetzung nach Anspruch 2, worin die Verbindung die folgende Strukturformel aufweist:

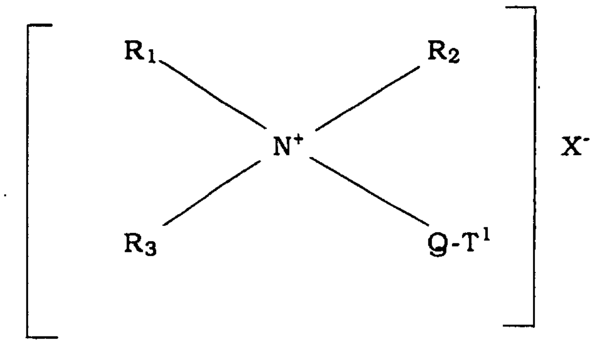

oder eine Aminvorstufe davon ist,
worin

$R_1$ Q'-$T^1$ oder Q'-$T^2$ oder $T^2$ oder $R_6$ ist,
$R_2$ Q"-$T^1$ oder Q"-$T^3$ oder $T^3$ oder $R_6$ ist,
Q

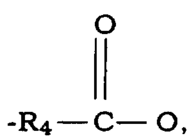 , 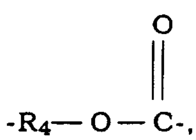 , oder 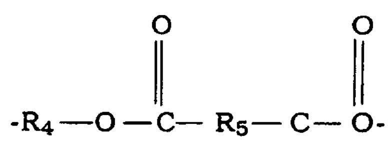

ist,
Q' und Q" wie Q definiert sind, worin jeweils $R_4$ $R_{4'}$ oder $R_{4''}$ ist und $R_5$ $R_{5'}$ oder $R_{5''}$ ist,
$R_3$ und $R_6$ dasselbe oder verschiedenes $C_1$-$C_4$-Alkyl oder $C_1$-$C_4$-Hydroxyalkyl oder H sind;
$R_4$, $R_{4'}$ und $R_{4''}$ sowie $R_5$, $R_{5'}$ und $R_{5''}$ irgendeine azyklische oder irgendeine mono- oder polyheterozyklische organische Gruppen sind,
$T^1$ der Wirksubstanzrest ist,
$T^2$ und $T^3$ dasselbe oder unterschiedliches $C_5$-$C_{30}$-Alkyl oder Alkenyl oder Aryl sind und $X^-$ ein kompatibles Anion darstellt.

4. Zusammensetzung nach Anspruch 3, worin $R_4$ $(CH_2)_n$ oder $(CH_2\text{-}CH_2\text{-}O)_nCH_2\text{-}CH_2\text{-}$ darstellt und $R_5$ $C_1$-$C_4$-Alkyl oder Alkenyl oder Aryl ist und n eine ganze Zahl zwischen 1 und 4 darstellt.

5. Zusammensetzung nach Anspruch 3, worin $R_4$ ein oder mehrere Hydroxy- und/oder Carboxy- und/oder Esterfunktionen enthält.

6. Zusammensetzung nach Anspruch 3, worin die Verbindung ist:

$$[\ N\ (Me)x\ (CH_2\text{-}CH_2\text{-}OOC\text{-}T_4)\ y\ (CH_2\text{-}CH_2\text{-}O\text{-}R_7)\ z]\ +\ X^-$$

oder

$$[\ N\ (Me)x\ ((CH_2)_n\text{-}COO\text{-}T_1)\ y\ ((CH_2)_n\text{-}COO\text{-}R_8)\ z]\ +\ X^-$$

oder

$$[\ N\ (Me)3\ -\ (CH_2\text{-}CH(OOC\text{-}T_4)\ (CH_2\text{-}O\text{-}R_9))]\ +\ X^-$$

oder

$$[\ N\ (Me)3\ -\ (CH_2\text{-}CH(OR_9)\ (CH_2\text{-}OOC\text{-}T_4))]\ +\ X^-$$

oder eine Aminvorstufe einer der obengenannten Verbindungen,
worin x+y+z = 4 ist
n eine ganze Zahl zwischen 1 und 4 ist
und $R_7$ H oder -OC-$T_2$ ist; $R_8$ H oder $T_2$ ist; $R_9$ $R_7$ oder -OC-$T_4$ ist,

T2 wie in Anspruch 3 definiert ist.

T4 T1 wie in Anspruch 3 definiert ist, oder einen Wirksubstanzrest darstellt, welcher durch Hydrolyse eine Wirksubstanz und eine Disäure bildet und

$X^-$ Chlorid oder MeSulfat darstellt.

**7.** Zusammensetzung nach Anspruch 1, umfassend eine Verbindung, enthaltend einen Stickstoff, worin das Stickstoffatom in der Verbindung Teil einer zyklischen Ammoniumeinheit und/oder einer Aminvorstufe dieser Verbindung oder einer Aminoxidgruppe ist.

**8.** Zusammensetzung nach Anspruch 7, worin die Verbindung die folgende Strukturformel aufweist:

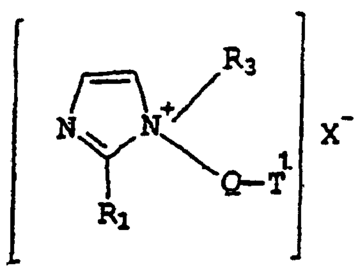 oder 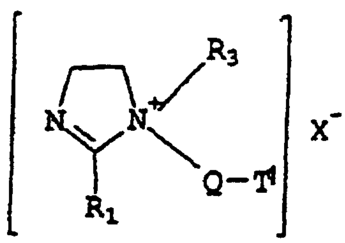 oder 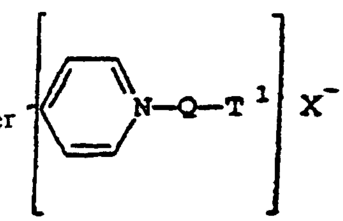

oder eine Aminvorstufe davon ist
oder

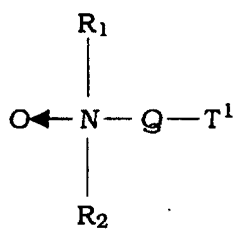

worin:

$R_1$ Q'-$T^1$ oder Q'-$T^2$ oder $T^2$ oder $R_6$ ist,
$R_2$ Q"-$T^1$ oder Q"-$T^3$ oder $T^3$ oder $R_6$ ist,
Q

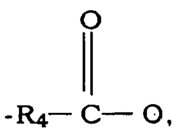, 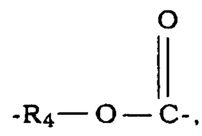 oder 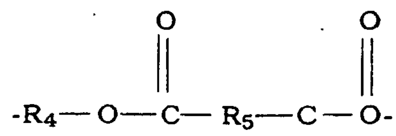

ist,

Q' und Q" wie Q definiert sind, worin jeweils $R_4$ $R_{4'}$ oder $R_{4''}$ darstellt und $R_5$ $R_{5'}$ oder $R_{5''}$ darstellt,

$R_3$ und $R_6$ dasselbe oder unterschiedliches $C_1$-$C_4$-Alkyl oder $C_1$-$C_4$-Hydroxyalkyl oder H sind;

$R_4$, $R_{4'}$ und $R_{4''}$ sowie $R_5$, $R_{5'}$ und $R_{5''}$ irgendeine azyklische oder irgendeine mono- oder polyheterozyklische organische Gruppe sind,

$T^1$ ein Wirksubstanzrest ist,

$T^2$ und $T^3$ dasselbe oder unterschiedliches $C_5$-$C_{30}$-Alkyl oder Alkenyl oder Aryl sind und $X^-$ ein kompatibles Anion darstellt.

**9.** Zusammensetzung nach Anspruch 8, worin $R_4$ $(CH_2)_n$ oder $(CH_2$-$CH_2$-$O)_nCH_2$-$CH_2$- ist und $R_5$ $C_1$-$C_7$-Alkyl oder Alkenyl oder Aryl ist und n eine ganze Zahl zwischen 1 und 4 darstellt.

**10.** Zusammensetzung nach Anspruch 1, umfassend eine Verbindung, enthaltend mehrere Stickstoffe, worin die Verbindung die folgende Strukturformel aufweist:

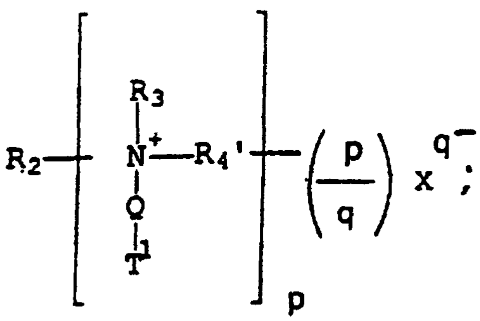 oder 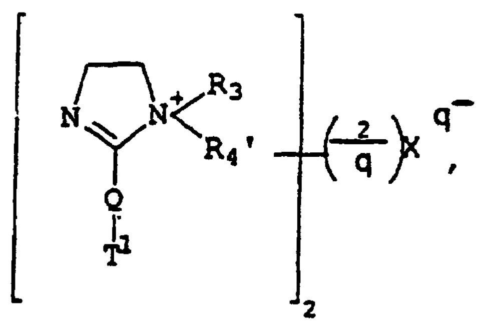

oder

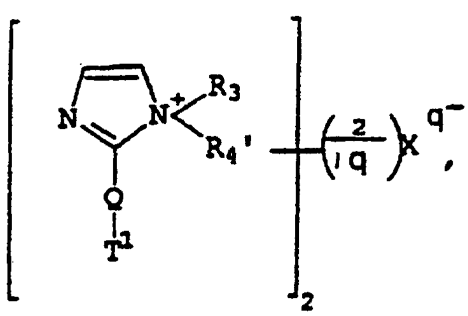 oder 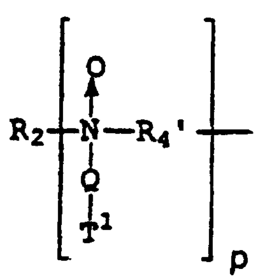

oder eine Aminvorstufe davon ist,

worin:

$R_2$ Q"-$T^1$ oder Q"-$T^3$ oder $T^3$ oder $R_6$ ist,

Q

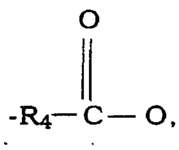 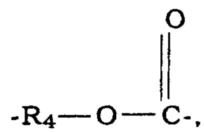 oder 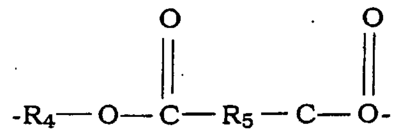

ist,
Q" wie Q definiert ist, worin $R_4$ $R_{4"}$ ist und $R_5$ $R_{5"}$ ist,
$R_3$ und $R_6$ dasselbe oder unterschiedliches $C_1$-$C_4$-Alkyl oder $C_1$-$C_4$-Hydroxyalkyl oder H sind,
$R_4$, $R_{4'}$ und $R_{4"}$, $R_5$ und $R_{5"}$ irgendeine azyklische oder irgendeine monooder polyheterozyklische organische Gruppe sind,
$T^1$ einen Wirksubstanzrest darstellt,
$T^3$ $C_5$-$C_{30}$-Alkyl oder Alkenyl oder Aryl ist und $X^-$ ein kompatibles Anion ist,
p und q ganze Zahlen sind.

**11.** Zusammensetzung nach Anspruch 10, worin $R_4$ $(CH_2)_n$ oder $(CH_2$-$CH_2$-$O)_nCH_2$-$CH_2$- ist und $R_5$ $C_1$-$C_7$-Alkyl oder Alkenyl oder Aryl ist und n eine ganze Zahl zwischen 1 und 4 darstellt.

**12.** Zusammensetzung nach Anspruch 1, worin die Verbindung, enthaltend Stickstoffatome, die folgende Strukturformel aufweist:

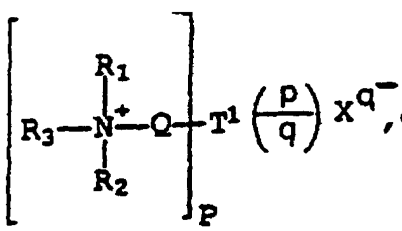 oder 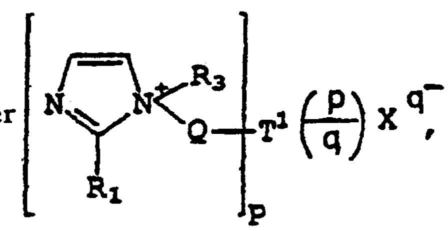

oder

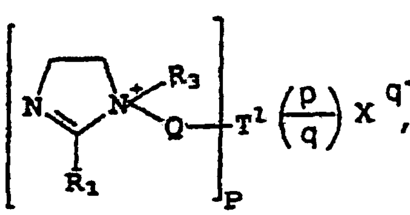 oder 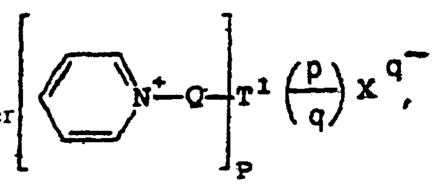 oder 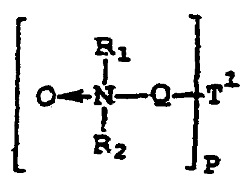

oder eine Aminvorstufe davon ist,
worin:

$R_1$ Q'-$T^1$ oder Q'-$T^2$ oder $T^2$ oder $R_6$ ist,
$R_2$ Q"-$T^1$ oder Q"-$T^3$ oder $T^3$ oder $R_6$ ist,
Q

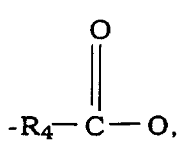 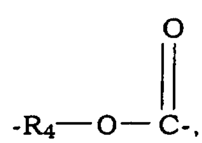 oder 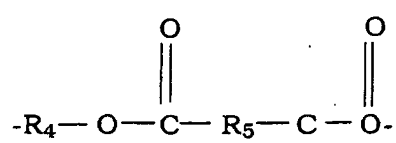

ist,
Q' und Q" wie Q definiert sind, worin jeweils $R_4$ $R_{4'}$ oder $R_{4"}$ darstellt und

$R_5$ $R_{5'}$ oder $R_{5''}$ darstellt,
$R_3$ und $R_6$ dasselbe oder unterschiedliches $C_1$-$C_4$-Alkyl oder $C_1$-$C_4$-Hydroxyalkyl oder H sind;
$R_4$, $R_{4'}$ und $R_{4''}$ sowie $R_5$, $R_{5'}$ und $R_{5''}$ irgendeine azyklische oder irgendeine mono- oder polyheterozyklische organische Gruppe sind,
$T^1$ ein Wirksubstanzrest ist,
$T^2$ und $T^3$ dasselbe oder unterschiedliches $C_5$-$C_{30}$-Alkyl oder Alkenyl oder Aryl sind und $X^-$ ein kompatibles Anion darstellt,
p eine ganze Zahl zwischen 1 und $\frac{MWT^1}{50}$ ist, bevorzugt zwischen $\frac{MWT^1}{1000}$ und $\frac{MWT^1}{100}$, und q eine ganze Zahl darstellt.

**13.** Zusammensetzung nach Anspruch 12, worin $R_4$ $(CH_2)_n$ oder $(CH_2\text{-}CH_2\text{-}O)_nCH_2\text{-}CH_2\text{-}$ ist und $R_5$ $C_1$-$C_7$-Alkyl oder Alkenyl oder Aryl ist und n eine ganze Zahl zwischen 1 und 4 darstellt.

**14.** Zusammensetzung nach Ansprüchen 3, 6, 8, 10 und 12, worin $T^2$ und $T^3$ dasselbe oder unterschiedliches $C_{12}$-$C_{22}$-Alkyl oder Alkenyl sind.

**15.** Zusammensetzung nach Anspruch 1, welche zusätzlich Gewebeweichmachermatrixkomponenten und -additive enthält, ausgewählt aus Silikon, schmutzabweisendem Mittel, organischem Lösungsmittel, nichtionischen Tensiden, Elektrolyten, Duftstoff, Konservierungsmitteln, Germiziden, Färbemitteln, Farbstoffen, Fungiziden, Stabilisatoren, Aufhellern, Trübungsmitteln, sowie Mischungen davon.

**16.** Zusammensetzung nach Anspruch 15, enthaltend zwischen 0.005% und 20,0% Wirksubstanzrest, bevorzugt zwischen 0,01% und 5,0% und ganz besonders bevorzugt zwischen 0,02% und 2,0% Wirksubstanzrest.

**17.** Zusammensetzung nach Anspruch 1, worin $X^-$ ein $C_7$-$C_{30}$-Alkyl- oder Alkenyl enthaltendes Anion ist.

**18.** Verwendung einer Zusammensetzung, umfassend ein weichmachendes Konditionierungsmittel und eine Verbindung, enthaltend einen Stickstoff, gebunden durch eine Esterbindung an einen Wirksubstanzrest, wobei der Wirksubstanzrest eine Wirksubstanz bildet, ausgewählt aus der Klasse der Säuren oder Alkohole natürlichen oder synthetischen Ursprungs, oder einer Verbindung nach Anspruch 15 in einer gewebeweichmachenden/konditionierenden Matrix als ein Duftstoffabgabesystem, als ein Biozidabgabesystem, als ein Fungizidabgabesystem, als ein Antitranspirationsmittelabgabesystem, als ein Insektizid- oder Insektenabwehrmittelsystem oder als ein Bügelhilfeabgabesystem.

**19.** Verwendung einer Zusammensetzung, umfassend ein weichmachendes Konditionierungsmittel und eine Verbindung, enthaltend einen Stickstoff, gebunden durch eine Esterbindung an einen Wirksubstanzrest, wobei der Wirksubstanzrest eine Wirksubstanz bildet, ausgewählt aus der Klasse der Säuren oder Alkohole natürlichen oder synthetischen Ursprungs, oder einer Verbindung nach Anspruch 1 in einer Reinigungsmittelmatrix als ein Duftstoff, Biozid, Fungizid, Antitranspirationsmittel, Insektizid oder Insektenabwehrmittel oder als ein Bügelhilfeabgabesystem.

**20.** Verwendung einer Zusammensetzung, umfassend ein weichmachendes Konditionierungsmittel und eine Verbindung, enthaltend einen Stickstoff, gebunden durch eine Esterbindung an einen Wirksubstanzrest, wobei der Wirksubstanzrest eine Wirksubstanz bildet, ausgewählt aus der Klasse der Säuren oder Alkohole natürlichen oder synthetischen Ursprungs, oder einer Verbindung nach Anspruch 1 als ein Duftstoffabgabesystem, ein Bakterizid- oder Insektizid-/Insektenabwehrmittelabgabesystem, ein Fungizidabgabesytem, ein Antitranspirationsmittelabgabesystem, ein Antischuppenmittelabgabesystem oder ein Korrosionsinhibitorabgabesystem.

## Revendications

**1.** Composition comprenant un agent conditionneur adoucissant et un composé contenant un azote lié par une liaison ester à un radical $T^1$ de substance active,

dans laquelle le radical de substance active produit une substance active choisie dans la classe des acides ou des alcools d'origine naturelle ou synthétique,

dans laquelle la substance active est active de telle sorte que la substance active soit efficacement déposée sur une surface et soit efficacement retenue sur ladite surface, puis libérée avec retard au cours du temps,

dans laquelle l'agent conditionneur adoucissant est choisi dans le groupe constitué par les constituants et

additifs de matrice d'adoucissant textile, les constituants et additifs de matrice de détergent, les constituants et additifs de matrice de shampooing, les constituants et additifs de matrice de conditionnement capillaire, les constituants et additifs de matrice de nettoyant pour surfaces dures, les constituants et additifs de matrice de parfum, les constituants et additifs de matrice de déodorant ou les constituants et additifs de matrice d'insecticide, et

dans laquelle $T^1$ produira une substance active choisie dans la classe des ingrédients de parfum à fonctions hydroxy et/ou carboxy, des bactéricides, des fongicides, des antitranspirants, de l'amidon ou de l'amidon modifié, des pesticides, des insecticides, des insectifuges et des inhibiteurs de corrosion.

**2.** Composition selon la revendication 1 dans laquelle le composé possède un azote présent dans un groupe ammonium quaternaire et/ou un précurseur amine de celui-ci.

**3.** Composition selon la revendication 2 dans laquelle le composé a la formule développée suivante :

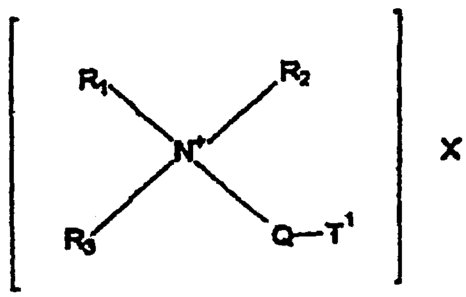

ou un précurseur amine de celui-ci
formule dans laquelle:

$R_1$ est Q'-$T^1$ ou Q'-$T^2$ ou $T^2$ ou $R_6$,
$R_2$ est Q"-$T^1$ ou Q"-$T^3$ ou $T^3$ ou $R_6$,
Q est

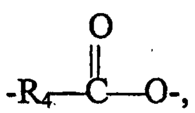, 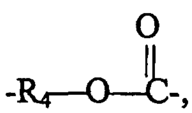, ou 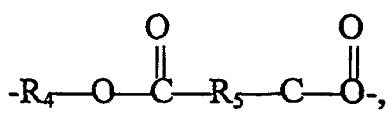,

Q' et Q" sont définis comme étant un radical Q dans lequel, respectivement, $R_4$ est $R_{4'}$ ou $R_{4''}$ et $R_5$ est $R_{5'}$ ou $R_{5''}$,
$R_3$ et $R_6$ peuvent être identiques ou différents et sont des groupes alkyle en $C_1$-$C_4$ ou hydroxyalkyle en $C_1$-$C_4$ ou H,
$R_4$, $R_{4'}$ et $R_{4''}$, et $R_5$, $R_{5'}$ et $R_{5''}$, sont des groupes organiques acycliques ou mono- ou poly-(hétéro)cycliques quelconques,
$T^1$ est le radical de substance active,
$T^2$ et $T^3$ sont des groupes alkyle ou alcényle ou aryle en $C_5$-$C_{30}$ (identiques ou différents), $X^-$ est un anion compatible.

**4.** Composition selon la revendication 3, dans laquelle $R_4$ est un groupe $(CH_2)_n$ ou $(CH_2\text{-}CH_2\text{-}O)_nCH_2\text{-}CH_2$- et $R_5$ est un groupe alkyle ou alcényle ou aryle en $C_1$-$C_4$ et n est un nombre entier compris entre 1 et 4.

**5.** Composition selon la revendication 3, dans laquelle $R_4$ contient une ou plusieurs fonctions hydroxy et/ou carboxy et/ou ester.

**6.** Composition selon la revendication 3, dans laquelle le composé est :

$$[N(Me)_x(CH_2\text{-}CH_2\text{-}OOC\text{-}T_4)_y(CH_2\text{-}CH_2\text{-}O\text{-}R_7)_z]^+ X^-$$

ou

$$[N(Me)_x((CH_2)_n\text{-}COO\text{-}T_1)_y((CH_2)_n\text{-}COO\text{-}R_8)_z]^+ X^-$$

ou

$$[N(Me)_3(CH_2\text{-}CH(OOC\text{-}T_4)(CH_2\text{-}O\text{-}R_9))]^+ X^-$$

ou

$$[N(Me)_3(CH_2\text{-}CH(OR_9)(CH_2\text{-}OOC\text{-}T^4))]^+ X^-$$

ou un précurseur amine d'un des composés ci-dessus,
où x+y+z = 4
n est un nombre entier compris entre 1 et 4
et $R_7$ est H ou $-OC-T_2$, $R_8$ est H ou $T_2$, $R_9$ est $R_7$ ou $-OC-T_4$,
$T^2$ est tel que défini dans la revendication 3,
$T^4$ est $T^1$ tel que défini dans la revendication 3, ou est un radical de substance active qui, par hydrolyse, produit une substance active et un diacide , et
$X^-$ est un chlorure ou un méthylsulfate.

**7.** Composition selon la revendication 1 comprenant un composé contenant un azote, dans lequel l'atome d'azote dans ledit composé fait partie d'un groupement ammonium cyclique et/ou d'un précurseur amine dudit composé ou d'un groupe oxyde d'amine.

**8.** Composition selon la revendication 7, dans laquelle le composé a la formule développée suivante:

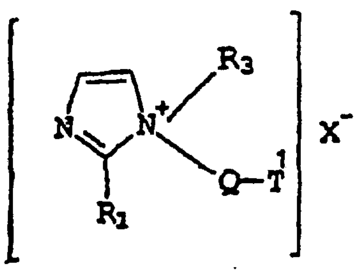 ou 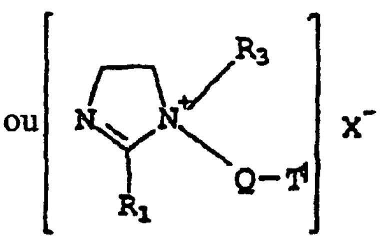 ou 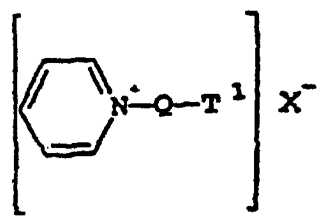

ou un précurseur aminé de celui-ci, ou

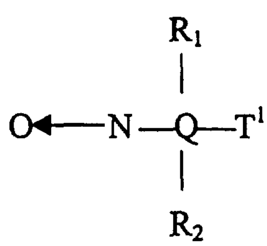

formules dans lesquelles:

$R_1$ est $Q'-T^1$ ou $Q'-T^2$ ou $T^2$ ou $R_6$,
$R_2$ est $Q''-T^1$ ou $Q''-T^3$ ou $T^3$ ou $R_6$,
Q est

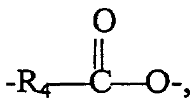, 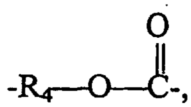, ou 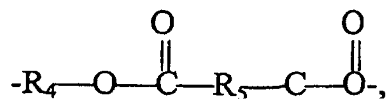,

Q' et Q" sont définis comme étant un radical Q dans lequel, respectivement, $R_4$ est $R_{4'}$ ou $R_{4''}$ et $R_5$ est $R_{5'}$ ou $R_{5''}$,

$R_3$ et $R_6$ peuvent être identiques ou différents et sont des groupes alkyle en $C_1$-$C_4$ ou hydroxyalkyle en $C_1$-$C_4$ ou H,

$R_4$, $R_{4'}$ et $R_{4''}$, et $R_5$, $R_{5'}$ et $R_{5''}$, sont des groupes organiques acycliques ou mono- ou poly-(hétéro)cycliques quelconques,

$T^1$ est un radical de substance active,

$T^2$ et $T^3$ sont des groupes alkyle ou alcényle ou aryle en $C_5$-$C_{30}$ (identiques ou différents), $X^-$ est un anion compatible.

**9.** Composition selon la revendication 8, dans laquelle $R_4$ est $(CH_2)_n$ ou $(CH_2\text{-}CH_2\text{-}O)_nCH_2\text{-}CH_2\text{-}$ et $R_5$ est un groupe alkyle ou alcényle ou aryle en $C_1$-$C_7$ et n est un nombre entier compris entre 1 et 4.

**10.** Composition selon la revendication 1, comprenant un composé contenant plusieurs atomes d'azote dans laquelle le composé a la formule développée suivante:

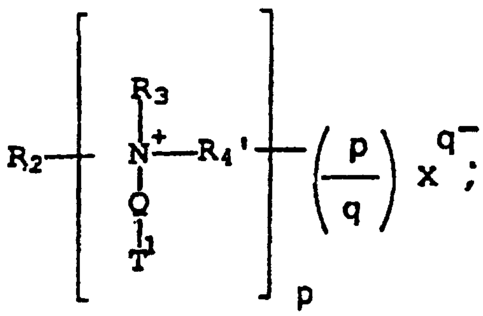 ; ou 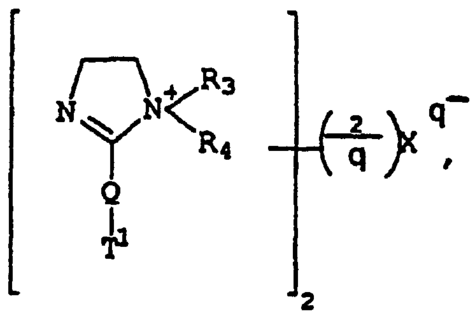

ou

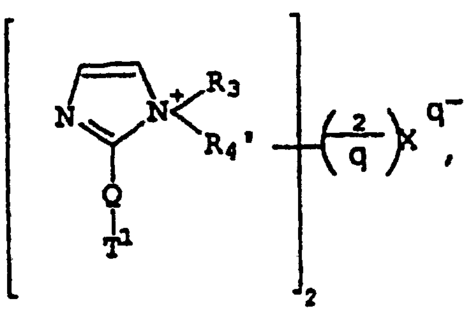 ou or 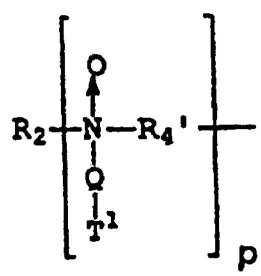

ou un précurseur aminé de celui-ci,

formules dans lesquelles:

$R_2$ est Q"-$T^1$ ou Q"-$T^3$ ou $T^3$ ou $R_6$,

Q est

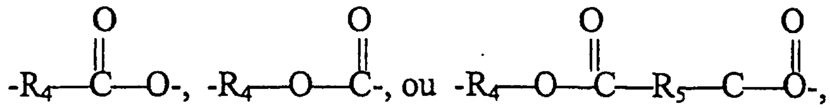

Q" est défini comme étant un radical Q dans lequel $R_4$ est $R_{4"}$ et $R_5$ est $R_{5"}$,
$R_3$ et $R_6$ peuvent être identiques ou différents et sont des groupes alkyle en $C_1$-$C_4$ ou hydroxyalkyle en $C_1$-$C_4$ ou H,
$R_4$, $R_{4'}$ et $R_{4"}$, et $R_5$, $R_{5'}$ et $R_{5"}$, sont des groupes organiques acycliques ou mono- ou poly-(hétéro)cycliques quelconques,
$T^1$ est un radical de substance active,
$T^3$ est un groupe alkyle ou alcényle ou aryle en $C_5$-$C_{30}$, $X^-$ est un anion compatible,
p et q sont des nombres entiers.

**11.** Composition selon la revendication 10, dans laquelle $R_4$ est $(CH_2)_n$ ou $(CH_2\text{-}CH_2\text{-}O)_nCH_2\text{-}CH_2\text{-}$ et $R_5$ est un groupe alkyle ou alcényle ou aryle en $C_1$-$C_7$ et n est un nombre entier compris entre 1 et 4.

**12.** Composition selon la revendication 1, dans laquelle le composé contenant des atomes d'azote a la formule développée suivante:

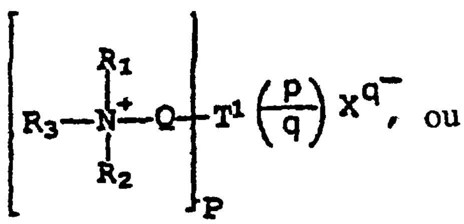
ou
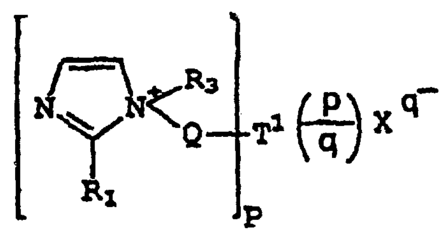

ou

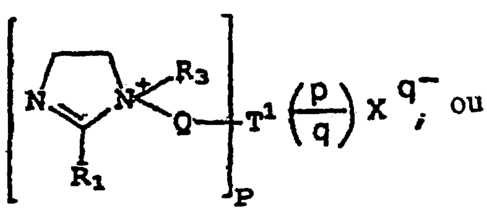
ou
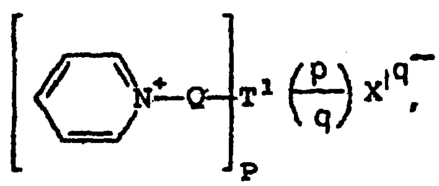
ou
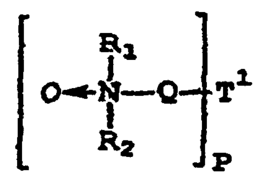

ou un précurseur amine de celui-ci,
formules dans lesquelles:

$R_1$ est $Q'\text{-}T^1$ ou $Q'\text{-}T^2$ ou $T^2$ ou $R_6$,
$R_2$ est $Q"\text{-}T^1$ ou $Q"\text{-}T^3$ ou $T^3$ ou $R_6$,
Q est

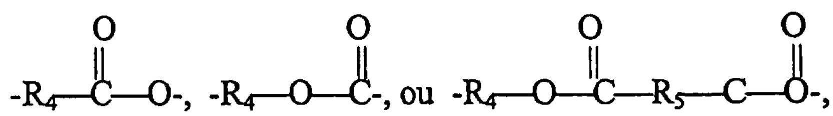

Q' et Q" sont définis comme étant un radical Q dans lequel, respectivement, $R_4$ est $R_{4'}$ ou $R_{4"}$ et $R_5$ est $R_{5'}$ ou $R_{5"}$,

$R_3$ et $R_6$ peuvent être identiques ou différents et sont des groupes alkyle en $C_1$-$C_4$ ou hydroxyalkyle en $C_1$-$C_4$ ou H,

$R_4$, $R_{4'}$ et $R_{4''}$, et $R_5$, $R_{5'}$ et $R_{5''}$, sont des groupes organiques acycliques ou mono- ou poly-(hétéro)cycliques quelconques,

$T^1$ est un radical de substance active,

$T^2$ et $T^3$ sont des groupes alkyle ou alcényle ou aryle en $C_5$-$C_{30}$ (identiques ou différents), $X^-$ est un anion compatible,

p est un nombre entier compris entre 1 et $MMT^1/50$, de préférence entre $MMT^1/1\,000$ et $MMT^1/100$ et q est un nombre entier.

**13.** Composition selon la revendication 12, dans laquelle $R_4$ est $(CH_2)_n$ ou $(CH_2\text{-}CH_2\text{-}O)_nCH_2\text{-}CH_2\text{-}$ et $R_5$ est un groupe alkyle ou alcényle ou aryle en $C_1$-$C_7$ et n est un nombre entier compris entre 1 et 4.

**14.** Composition selon les revendications 3, 6, 8, 10 et 12, dans laquelle $T^2$ et $T^3$ sont des groupes alkyle ou alcényle en $C_{12}$-$C_{22}$ (identiques ou différents).

**15.** Composition selon la revendication 1 qui contient, en outre, des constituants et additifs pour matrice d'adoucissant textile choisis parmi une silicone, un agent de libération des salissures, un solvant organique, des non ioniques, un électrolyte, un parfum, des conservateurs, des germicides, des colorants, des teintures, des fongicides, un stabilisant, un azurant, un opacifiant, et leurs mélanges.

**16.** Composition selon la revendication 15, contenant entre 0,005% et 20,0% de radical de substance active, de préférence entre 0,01% et 5,0% et tout particulièrement entre 0,02% et 2,0% de radical de substance active.

**17.** Composition selon la revendication 1, dans laquelle $X^-$ est un anion contenant un groupe alkyle ou alcényle en $C_7$-$C_{30}$.

**18.** Utilisation d'une composition comprenant un agent conditionneur adoucissant et un composé contenant un azote lié par une liaison ester à un radical de substance active, le radical de substance active produisant une substance active choisie dans la classe des acides ou des alcools d'origine naturelle ou synthétique ; ou d'une composition selon la revendication 15, dans une matrice d'adoucissant/conditionneur textile comme système de délivrance d'un parfum, comme système de délivrance d'un biocide, comme système de délivrance d'un fongicide, comme système de délivrance d'un antitranspirant, comme système insecticide ou insectifuge ou comme système de délivrance d'un auxiliaire de repassage.

**19.** Utilisation d'une composition comprenant un agent conditionneur adoucissant et un composé contenant un azote lié par une liaison ester à un radical de substance active, le radical de substance active produisant une substance active choisie dans la classe des acides ou des alcools d'origine naturelle ou synthétique ; ou d'une composition selon la revendication 1 dans une matrice de détergent comme parfum, biocide, fongicide, antitranspirant, insecticide ou insectifuge, ou comme système de délivrance d'un auxiliaire de repassage.

**20.** Utilisation d'une composition comprenant un agent conditionneur adoucissant et un composé contenant un azote lié par une liaison ester à un radical de substance active, le radical de substance active produisant une substance active choisie dans la classe des acides ou des alcools d'origine naturelle ou synthétique ; ou d'une composition selon la revendication 1 comme système de délivrance d'un parfum, système de délivrance d'un bactéricide ou d'un insecticide/insectifuge, système de délivrance d'un fongicide, système de délivrance d'un antitranspirant, système de délivrance d'un antipelliculaire ou système de délivrance d'un inhibiteur de corrosion.